# EUROPEAN PATENT SPECIFICATION

(11) **EP 0 488 999 B1**
(45) Date of publication and mention of the grant of the patent: **09.07.1997**
(21) Application number: 92200537.6
(22) Date of filing: 16.04.1987
(51) Int. Cl.: C12P 41/00, C12P 7/40

(54) **A method for producing optically active cyclopropane carboxylic acid**
Verfahren zur Herstellung von optisch-aktiven Cyclopropancarbonsäure
Méthode de production d'acides cyclopropane-carboxyliques optiquement actifs

(30) Priority: 16.04.1986 JP 89803/86; 24.04.1986 JP 95444/86
(43) Date of publication of application: 03.06.1992
(62) Divisional of application: 87902734.0
(73) Proprietor: SUMITOMO CHEMICAL COMPANY LIMITED, Osaka-shi, Osaka 541 (JP)
(72) Inventor: Nishizawa, Kanji, Takarazuka-shi, Hyogo 665 (JP); Mitsuda, Satoshi, Takarazuka-shi, Hyogo 665 (JP); Komaki, Ryohei, Misawa-shi, Aomori 033 (JP); Nishizawa,Masako, Takarazuka-shi, Hyogo 665 (JP); Sugiki, Chiaki, Ibaraki-shi, Osaka 567 (JP); Ogami, Yasutaka, Higashinada-ku, Kobe-shi, Hyogo 658 (JP); Sonoda, Kazumi, Amagasaki-shi, Hyogo 661 (JP); Kishimoto, Fumitaka, Kawanishi-shi, Hyogo 666-01 (JP)
(74) Representative: Cresswell, Thomas Anthony

(56) References cited:
- EP-A- 0 164 573
- CHEMICAL ABSTRACTS, vol. 102, no. 23, June 1985, Columbus, Ohio, US; abstract no. 202608Q, SUMITOMO: 'Biochemical production of optically active chrysanthemumic acid esters' page 490 ;
- CHEMICAL ABSTRACTS, vol. 104, no. 21, May 1986, Columbus, Ohio, US; abstract no. 184884, MITSUTA,MASARU ET AL.: 'Biochemical preparation of optically active chrysanthemic acid' page 514 ;

## Description

The present invention relates to a method for producing an optically active cyclopropane carboxylic acid. More particularly, the present invention relates to a method for producing the (+)-trans optically active cyclopropane carboxylic acid or its salts represented by the following formula (I): 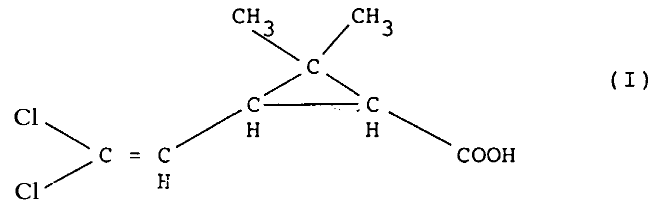 by asymmetric hydrolysis of cyclopropane carboxylic acid esters represented by the following formula (II): 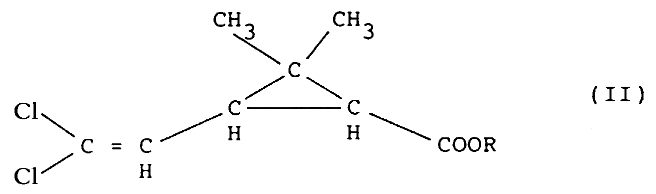 wherein R is as defined below, with microorganisms or esterases produced by the microorganisms.

The cyclopropane carboxylic acids represented by the above formula (I) constitutes the acid moiety of a low toxic and rapidly acting insecticidal ester generally termed pyrethroid such as allethrin, permethrin, decamethrin, teffuluthrin, etc.

The cyclopropane carboxylic acids represented by the formula (I) contain two asymmetric carbon atoms at the 1-position and the 3-position and therefore have four diastereomers. Of these isomers, those having the absolute configurations of "1R, 3S" and "1R and 3R" are termed "(+)-trans isomer" and "(+)-cis isomer" (according to RS nomenclature),.respectively, because the optical rotation of these isomers is (+) in a specific solvent and substitution groups of them are in a trans-form and a cis-form, respectively. And, the other two isomers having the absolute configurations of "1S, 3S" and "1S, 3R" are termed "(-)-trans isomer" and "(-)-cis isomer", respectively, because the optical rotation of these isomers is (-) in a specific solvent and substitution group of them are in a trans-form and a cis-form, respectively. Only the (+)-isomers have insecticidal activities as pyrethroid esters and the (-)-isomers have almost no insecticidal activities as pyrethroids.

The relative effects of the cis and the trans isomers vary according to the kinds of harmful insects to be killed and the type of effect.

It is possible to use the (+)-trans-pyrethroidal compound and (+)-cis- compound independently for different purposes. Accordingly, the production of (+)-cyclopropane carboxylic acids in effective manner is industrially important.

The presently known major method for production of (+)-isomers is organosynthetic optical resolution, but development of more economical methods for optical resolution is now desired for production of (+)-isomers because the organosynthetic optical resolution requires a relatively expensive optically active reagent or a complicated step. There are known methods for producing optically active (+)-trans acid by resolving cyclopropane carboxylates by asymmetric hydrolysis with an pig liver esterase (e.g., Schneider et al., Angewande chemie International Edition in English 23. 64 (1984)) or with a microbial esterase (Japanese Patent Publication (Kokai) No. 244295/1985).

However, the former method is industrially disadvantageous because of the expensiveness and only limited supply of the pig liver esterase. As for the latter method, it is reported that the trans-isomer of cyclopropane carboxylic acid ester is preferentially resolved by asymmetric hydrolysis but the method can not be industrially applied because the yield of (+)-trans-cyclopropane carboxylic acid is as low as 31mg/100ml culture medium, and the concentration of the substrate and the optical purity of the resulting (+)-trans cyclopropane carboxylic acid are low.

Under these circumstances, the inventors of this invention continuously studied in order to develop an industrially advantageous method for producing (+)-cyclopropane carboxylic acid (I).

It was found that (±)-cis,trans-2,2-dimethyl-3(2,2-dichlorovinyl)cyclopropane carboxylic acid esters represented by formula (II): 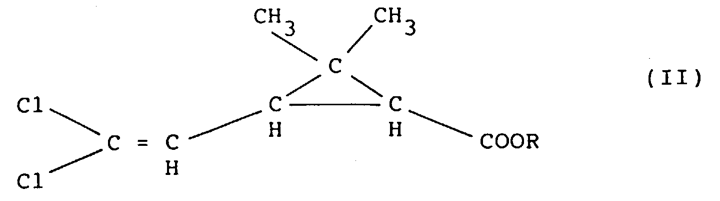 wherein R represents alkyl group containing 1-4 carbon atom or alkyl group containing 1-4 carbon atom substituted by halogen atom, which does not indicate the configuration of the esters, are asymmetrically hydrolyzed effectively into (+)-trans-2,2-dimethyl-3(2,2-dichlorovinyl)-cyclopropane carboxylic acid and esters of its stereoisomers with the following microorganisms or esterase produced by the microorganisms:

| | |
|---|---|
| Arthrobacter globiformis | IFO-12958 |
| Thermomyces lanuginosus | IFO-9863 |
| Rhodotorula rubra | IFO-0918 |
| Rhodotorula rubra | IFO-1100 |
| Rhodotorula rubra | IFO-0889 |
| Rhodotorula rubra | IFO-0909 |
| Candida humicola | IFO-0760 |
| Candida lipolytica | NRRL-Y-6795 |
| Aspergillus oryzae | ATTC-14605 |
| Aspergillus flavus | ATTC-11492 and |
| Bacillus sp. DC-1 (BP-FERM 1254) | |

and that (+)-trans-2,2- dimethyl-3(2,2-dichlorovinyl)cyclopropane carboxylic acid or its salts are prepared with high optical purity.

In particular, it was confirmed that when Bacillus sp. DC-1, which is a microorganism isolated by the present inventors, is used, (+)-trans-2,2-dimethyl-3(2,2-dichlorovinyl)cyclopropane carboxylic acid or its salts are obtained with high optical purities in 10 times or more (when compared in yields per 100ml of culture medium) the yields of conventional methods.

This invention provides a method for producing (+)-trans-2,2-dimethyl-3(2,2-dichlorovinyl)cyclopropane carboxylic acid or its salts which comprises allowing (±)-cis,trans-2,2-dimethyl-3(2,2-dichlorovinyl)cyclopropane carboxylic acid ester represented by the formula (II): 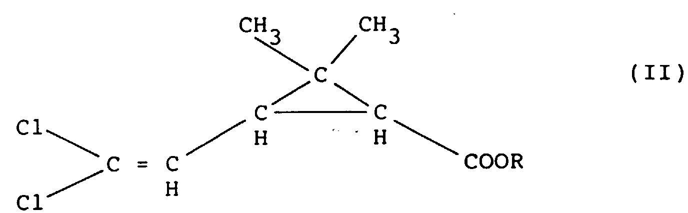 wherein R represents C₁-C₄alkyl group or C₁-C₄ alkyl group having halogen atom substituent, which does not indicate the configuration, to react with
Arthrobacter globiformis IFO-12958;
Thermomyces lanuginosus IFO-9863;
Rhodotorula rubra IFO-0918;
IFO-1100, IFO-0889, IFO-0909;
Candida humicola IFO-0760;
Candida lipolytica NRRL-Y-6795;
Aspergillus oryzae ATCC-14605;
Aspergillus flavus ATTC-11492; or
Bacillus sp. DC-1 (BP-FERM 1254)
or esterase produced by the above microorganisms until asymmetric hydrolysis is effected to produce (+)-trans-2, 2-dimethyl-3(2,2-dichlorovinyl)cyclopropane carboxylic acid and esters of its stereoisomers and then recovering the resulting (+)-trans-2,2-dimethyl-3(2,2-dichlorovinyl)-cyclopropane carboxylic acid or its salts.

The esters represented by the formula (II), which are starting materials in this invention, are easily obtained by well-known methods, for example, Pestic. Sci. Vol. 11, pp. 156-164 (1980). As the esters used as the starting meterials, methyl ester, ethyl ester, propyl ester, butyl ester, monochloroethyl ester, monochloropropyl ester, monobromoethyl ester, etc. are conveniently used. Especially, methyl ester, ethyl ester, and monochloroethyl ester are advantageous because of commercial availability and ease in handling.

Example of salts of cyclopropane carboxylic acids represented by the formula (I) is alkali metal salt such as sodium salt.

Cultivation of the microorganisms is carried out in the usual procedure. For example, microorganisms are cultured in a liquid medium, for instance, inoculating microorganisms in a sterilized liquid medium and then cultivating usually at 20-40°C, for 1-8 days with shaking. Alternatively, a solid medium may be used.

Any composition of the culture medium may be used, as long as the medium is familiar to cultivation of microorganisms and is utilizable by the microorganism used in this invention. As carbon sources and nitrogen sources in the medium, for example, glucose, starch, dextrin, molasses, fats and fatty oils, soybean powder, defatted soybean powder, fatty bean cake and corn steep liquor are employed. Ammonium sulfate, dipotassium hydrogenphosphate, magnesium sulfate and urea etc. may be used as inorganic salts in the medium. The cyclopropane carboxylic acid esters represented by the formula (II) or a fatty acid ester may be added to the medium.

In the method of this invention, asymmetric hydrolysis of cyclopropane carboxylic acid esters represented by the formula (II) is performed by mixing the esters (II) with a culture solution of the above microorganisms, a cell suspension, an esterase-containing aqueous solution such as a liquid esterase extract or its concentrated solution or their processed products, such as crude esterase, purified esterase etc., and then stirring or shaking the thus prepared mixture.

It is advantageous to perform the reaction at 10-65°C. Since the stability of the esterase is likely to decrease at high temperature and the reaction rate is small at low temperatures, it is preferable that the reaction temperature be within the range of 20-50°C. It is desirable that the pH of the mixture during reaction be 3-11, preferably around 5-10. It is preferable that buffers, such as phosphate buffer, be used to keep suitable pH during reaction.

Reaction time, which varies depending on the reaction conditions such as amount of esterase produced by the microorganisms, temperature during reaction, etc, is within the range of 2-3 to about 150 hours.

It is advantageous that the concentration of cyclopropane carboxylic acid esters represented by the formula (II) as a substrate when asymmetric hydrolysis is effected be 0.1-50 wt%, preferably 1-25 wt% based on a reaction liquid.

To the reaction mixture, 0.01-1 % of surface active agents such as Toriton X-100, Tween 80 or Brij 58 may be added, if necessary.

Cells of the microorganisms or the esterases may be used in the immobilized form, which is prepared by immobilizing them in a usual manner on inorganic or organic carriers, for example, zeolite, alumina, polysaccharide, polyamide, polystyrene resin, polyacrylic resih, and polyurethane resin, etc.

Cell suspension, suspension of ground cells or aqueous solution containing esterase, are prepared according to the usual method. Cell suspension is prepared by separating the cells harvested from culture medium by centrifugation or ultra filtration and suspending the cells in distilled water, ion-exchanged water, or buffer solution containing inorganic or organic salts, such as phosphate buffer solution. Suspension of ground cells is prepared by applying ultrasonic treatment, high pressure-breaking treatment by Manton-Gaulinhomogenizer or French Press or lytic enzyme treatment to the cell suspension. If necessary, the suspension may be changed to crude esterase solution by removing ground cell residue from the above suspension using centrifugation or ultrafiltration. Esterase is obtained from the suspension of ground cells by a conventional method, such as salting out with ammonium sulfate or sodium sulfate; or precipitation with organic solvents using hydrophylic organic solvents such as ethanol, propylalcohol, acetone, etc. Aqueous solution containing esterase is prepared by dissolving this obtained esterase in distilled water, ion-exchanged water, or buffer containing inorganic or organic salts, for example, phosphate buffer solution.

After the asymmetric hydrolysis is over, the liberated optically active cyclopropane carboxylic acid derivative represented by the formula (I) is separated from the unaltered ester and recovered by extraction with a solvent, column chromatography and fractional distillation, etc. For example, the reaction mixture is subjected to extraction with an organic solvent such as methyl isobutyl ketone, chloroform, ether, benzene or toluene and then the extract is subjected to fractional distillation under reduced pressure to separate the liberated optically active cyclopropane carboxylic acid derivatives represented by the formula (I) from the unaltered esters.

One of microorganisms utilized in this invention is Bacillus sp. DC-1, which is a novel microorganism, the characteristics of which are as follows.
(a) Morphological characteristics
1) From and size of cell:
   Rod, (0.5 - 0.6) µm x (1.2 - 1.7) µm.
   Occuring singly or in short chains.
2) Polymorphism: None
3) Motility: Motile by peritrichous flagella.
4) Spore formation: Endospores formed. Spherical or slightly oval with 0.4 - 0.6 µm in diameter. Spores formed in a terminal position of the vegetative cell, having swell.
5) Gram staining: Negative
6) Acid fastness: Negative

(b) Cultural characteristics on various mediums
1) Bouillon agar plate (35°C, 24 hours)
   Shape: Circular and projected form
   Margine: None
   Surface: Smooth and lustrous
   Color tone: Translucent and yellowish white
2) Bouillon agar slant (35°C, 24 hours)
   Growth degree: Moderate, growing like spread cloth or beads-like
   Surface: Smooth and lustrous
   Color tone: Translucent and yellowish white
3) Bouillon liquid (35°C, 24 hours)
   Growth degree: Moderate
   Coloring / discoloring: None
   Pellicle: Not formed
   Sediment: Formed
4) Bouillon gelatine stab (35°C, 14 days)
   No liquefaction.
5) Litmus milk (35°C, 14 days)
   Slightly alkaline. No coagulation nor peptonization.

(c) Physiological characteristics:
Cultured at 35°C for 1 - 5 days. Negative ones were observed up to 14 days.
1) Reduction of nitrate: Positive
Nitrite produced from nitrate.
2) Denitrification: Negative
3) MR test: Negative
4) VP test: Negative
5) Production of indole: Negative
6) Production of hydrogen sulfide: Negative
7) Hydrolysis of starch: Negative
8) Utilization of citric acid:
Koser medium: Negative
Christensen medium: positive

9) Utilization of inorganic nitrogen sources:
Stanier et al's medium modified by
Yamazato et al: (Yamazato et al. J. Gen. Appl.
Microbiol. (1982) 28:195-213)
Sodium succinate was used as the sole carbon source.
Nitrate: Not utilized
Ammonium salt: Utilized.

10) Pigmentation: Negative
11) Urease:
Christensen urea medium: Positive
12) Oxidase: Positive
13) Catalase: Positive
14) Range of growth:
Growth temperature: 10-45°C (optimum 30-35°C)
Growth pH: 6.0-9.5 (optimum 8.5-9.0)

15) Anaerobic or aerobic growth: Aerobic
16) OF test: Negative
17) Production of acid or gas from saccharides:

| | | Acid | Gas |
|---|---|---|---|
| (1) | L-arabinose | - | - |
| (2) | D-xylose | - | - |
| (3) | D-glucose | - | - |
| (4) | D-mannose | - | - |
| (5) | D-fructose | - | - |
| (6) | D-galactose | - | - |
| (7) | Maltose | - | - |
| (8) | Sucrose | - | - |
| (9) | Lactose | - | - |
| (10) | Trehalose | - | - |
| (11) | D-sorbitol | - | - |
| (12) | D-mannitol | - | - |
| (13) | Inositol | - | - |
| (14) | Glycerin | - | - |
| (15) | Starch | - | - |

Reference is made to Bergey's Manual of Determinative Bacteriology, 8th Ed (1974). The strain is identified as that belonging to genus Bacillus, since it is able to grow under aerobic conditions and forms endospores. The present strain is close to but different from Bacillus sphaericus and Bacillus pasteurii, as shown in the table below:

| | the Present strain | Bacillus sphaericus | Bacillus pasteurii |
|---|---|---|---|
| Reduction of nitrate | + | - | - |
| Requirement of urea or ammonia | - | - | + |

In view of the above facts, the present inventors have recognized the strain to be a novel one belonging to genus Bacillus and nominated it Bacillus sp. DC-1. The strain was deposited with Fermentation Research Institute, Industrial Technology Agency, Japan (Address: 1-3, Higashi 1-chome, Yatabe-cho, Tukuba-Gun, Ibaragi, JAPAN) under FERM BP-1254. The strain was first deposited as FERM P-8719 on March 31, 1986, then renumbered as FERM BP-1254 under Budapest Treaty on January 12, 1987.

This invention will be explained more precisely according to the following examples. However, this invention is not restricted to these examples and usual modifications or improvements of these are within the scope of the present invention.

### Example 1

After 30 g of soluble starch, 7 g of polypeptone, 5 g of yeast extract and 5 g of potassium dihydrogenphosphate were dissolved in 1 ℓ of distilled water, the pH of the solution was adjusted to 5.0 with 6N hydrochloric acid. After 10 ml of the above liquid medium was put in a test tube of 24 mm diameter, which was then plugged with cotton, it was sterilized at 120°C under high pressure for 15 minutes a loopful of cells of Arthrobacter globiformis IFO-12958 was inoculated into the medium and was subjected to shaking culture at 30°C for 24 hours to prepare a seed culture. After 300 ml of a liquid medium which has the same composition as above was put in a Sakaguchi's flask of 2 ℓ capacity and sterilized in the same manner as above, 5 ml of the seed culture prepared as above was inoculated into the sterilized liquid medium and was subjected to shaking culture at 30°C for 30 hours. After that, the resulting cultured solution was centrifuged to obtain 5 g (wet weight) of cells which were then suspended in 20 ml of 0.3M NaOH-Na₂CO₃ buffer solution (pH 10). To the suspension was added 1.0 g of ethyl (±)-cis, trans-2,2-dimethyl-3(2,2-dichlorovinyl)cyclopropane carboxylate (cis/trans ratio = 45/55) and the mixture was allowed to react with stirring at 50°C for 48 hours. Two ml of 35% hydrochloric acid was added to the reaction solution and the resulting mixture was subjected to extraction with 50 ml of methyl isobutyl ketone.

The extract was then analyzed by gas chromatography (column: Shinchrom F-51 (5%) + H₃PO₄ (1%), 2.6 m, 185°C) and the yield of 2,2-dimethyl-3(2,2-dichlorovinyl)cyclopropane carboxylic acid was calculated from the ratio of the peak area of this compound to that of ethyl 2,2-dimethyl-3(2,2-dichlorovinyl)cyclopropane carboxylate. All of the starting ethyl 2,2-dimethyl-3-(2,2-dichlorovinyl)cyclopropane carboxylate were recovered as it was excluding that which had been converted to 2,2-dimethyl-3(2,2-dichlorovinyl)cyclopropane carboxylic acid. IN sodium hydroxide solution was added to the extract to transfer 2,2-dimethyl-3(2,2-dichlorovinyl) cyclopropane carboxylic acid as a sodium salt into an aqueous layer. The pH of the aqueous layer was adjusted to 2 or lower with hydrochloric acid to liberate 2,2-dimethyl-3(2,2-dichlorovinyl)cyclopropane carboxylic acid and this compound was extracted with methyl isobutyl ketone. The extract was concentrated and evaporated to dryness to obtain almost chemically pure 2,2-dimethyl-3(2,2-dichlorovinyl)cyclopropane carboxylic acid.

After 5 mg of thus obtained 2,2-dimethyl-3(2,2-dichlorovinyl)cyclopropane carboxylic acid was dissolved in 1 ml of toluene, equal molar amount each of thionyl chloride, pyridine and 3,5-dichloroaniline were added and allowed to react to produce anilide. The relative concentrations of isomers of 2,2-dimethyl-3(2,2-dichlorovinyl)cyclopropane carboxylic acid were determined by analyzing the resulting anilide by high performance liquid chromatography (column: SUMIPAX OA 2100, moving phase: n-hexane/dichloroethane=17/3, flow rate:1.0 ml/minute). The results are shown in the following Table.

The hydrolysis rate shown in the table represents the molar ratio of the resulting 2,2-dimethyl-3(2,2-dichlorovinyl)cyclopropane carboxylic acid to ethyl (±)-cis, trans-2,2-dimethyl-3(2,2-dichlorovinyl)cyclopropane carboxylate used as the starting material.

| Hydrolysis rate (%) | Ratio of the isomers of the resulting 2,2-dimethyl-3(2,2-dichlorovinyl)cyclopropane carboxylic acid |
|---|---|
| | (+)-cis isomer/(-)-cis isomer/ (+)-trans isomer/(-)-trans isomer |
| 22.8 | 0:0:100:0 |

### Example 2

A medium was prepared by dissolving 20 g of glucose, 50 g of corn steep liquor, 2 g of potassium dihydrogenphosphate, 1 g of magnesium sulfate heptahydrate, 5 g of calcium carbonate and 5 g of ethyl butyrate in 1 ℓ of distilled water, the pH of which was adjusted to 6.0 with 6N hydrochloric acid. After 10 ml of the above liquid medium was put in a test tube and sterilized in the same manner as Example 1, a loopful of cells of Thermomyces lanuginosus IFO-9863 was inoculated into the sterilized medium and was subjected to shaking culture at 45°C for 48 hours to give a seed culture. After 300 ml of a liquid medium which was the same composition as above was put in a Sakaguchi's flask of 2 ℓ capacity and sterilized in the same manneer as above, 8ml of the seed culture prepared as above was inoculated into the sterilized liquid medium and was subjected to shaking culture at 45°C for 48 hours. The resulting cultured solution was centrifuged to collect cells which were then washed with 50ml of distilled water. To the washed cells was added 50 ml of 0.1M phosphate buffer solution (pH 8.0) and they were ground by ultrasonic treatment.

After the resulting liquid solution containing ground cells was centrifuged and the supernatant was collected, it was concentrated three times using an ultrafilter. To the concentrated supernatant was added 1.0 g of ethyl (±)-cis, trans-2,2-dimethyl-3(2,2-dichlorovinyl)cyclopropane carboxylate (cis/trans ratio = 45/55) and the mixture was allowed to react with stirring at 40°C for 72 hours. Following that, the same operation as used in Example 1 was performed and the following results were obtained.

| Hydrolysis ratio | Ratio of the isomers of the resulting 2,2-dimethyl-3(2,2-dichlorovinyl) cyclopropane carboxylic acid |
|---|---|
| (%) | (+)-cis-isomer/(-)cis-isomer/ (+)-trans-isomer/(-)-trans-isomer |
| 9.5 | 0 : 0 : 96.7 : 3.3 |

### Example 3

After 2 ℓ of a liquid medium having the same composition as the one used in Example 1 was put in a small fermentor and sterilized at 120°C under high pressure for 15 minutes, 100 ml of a seed culture of Arthrobacter globiformis IFO-12958 prepared in the same manner as Example 1 was inoculated into the sterilized liquid medium and was subjected to airation agitation culture at 30°C for 24 hours. Following that, the cultured solution was centrifuged to harvest 53 g (wet weight) of cells. After the harvested cells were suspended in 200 ml of 0.3 M NaOH-Na₂CO₃ buffer solution (pH 10), the cells were ground using a French press (product of the American Amico Company) and the ground cells were removed by centrifugation to obtain a crude enzyme solution. The crude enzyme solution was then subjected to ammonium sulfate fractionation to collect a 30-60% saturation fraction and this was lyophilized to obtain 1.3 g of a crude enzyme powder. After 0.5 g of the crude enzyme powder was dissolved in 20 ml of 0.1 M NaOH-Na₂CO₃ buffer solution (pH 10), 2.0 g of monochloroethyl (+)-cis,trans-2,2-dimethyl-3(2,2-dichlorovinyl)cyclopropane carboxylate (cis/trans ratio = 45/55) was added to the solution and the resulting mixture was allowed to react with stirring at 50°C for 17 hours. To the reaction mixture was added 2 ml of 35% hydrochloric acid and the mixture was subjected to extraction with 50 ml of methyl isobutyl ketone. After that, the same operation used in Example 1 was performed and the following results were obtained.

| Hydrolysis rate | Ratio of the isomers of the resulting 2,2-dimethyl-3(2,2-dichlorovinyl) cyclopropane carboxylic acid |
|---|---|
| (%) | (+)-cis isomer/(-)-cis isomer/ (+)-trans isomer/(-)-trans isomer |
| 18.0 | 11.1 : 0 : 88.9 : 0 |

### Example 4

A medium was prepared by dissolving 5 g of yeast extract, 5 g of polypeptone, 1 g of potassium dihydrogenphosphate and 0.2 g of magnesium sulfate (heptahydrate) in 1 ℓ of distilled water, the pH of which was adjusted to 9.0 with 10% sodium carbonate solution. After 10 ml of the above liquid medium put in a test tube of 24 mm diameter was sterilized with steam at 120°C under high pressure for 15 minutes, a loopful of cells of Bacillus sp. DC-1 was inoculated into the sterilized liquid medium and was subjected to shaking culture at 30°C for 24 hours to prepare a seed culture. After 100 ml of a liquid medium which has the same composition as above was put in an Erlenmeyer flask of 500 ml capacity and sterilized in the same manner as above, 1 ml of the seed culture prepared as above was inoculated with the sterilized liquid medium and was subjected to shaking culture at 30°C for 24 hours. To the cultured solution was added 1.5 g of ethyl (±)-cis,trans-2,2-dimethyl-3(2,2-dichlorovinyl)cyclopropane carboxylate (cis/trans ratio = 45/55) and the mixture was allowed to react with shaking at 30°C for 120 hours. To the resulting reaction solution was added 1 ml of 35% HCl solution and the resulting mixture was subjected to extraction with 50 ml of methyl isobutyl ketone. The extraction layer was analyzed by gas chromatography (column: 3% Thermon 3000, 1.1 m, 140°C) and the yield of 2,2-dimethyl-3-(2,2-dichlorovinyl)cyclopropane carboxylic acid was calculated from the ratio of the peak area of this compound to that of ethyl 2,2-dimethyl-3-(2,2-dichlorovinyl)cyclopropane carboxylate. All of the starting ethyl 2,2-dimethyl-3-(2,2-dichlorovinyl)cyclopropane carboxylate were recovered excluding that which had been converted to 2,2-dimethyl-3-(2,2-dichlorovinyl)cyclogropane carboxylic acid.

To the extract was added IN sodium hydroxide solution to transfer 2,2-dimethyl-3(2,2-dichlorovinyl)-cyclopropane carboxylic acid as sodium salt into the aqueous layer. The pH of the aqueous layer was adjusted to 2 or lower with HCl solution to liberate 2,2-dimethyl-3(2,2-dichlorovinyl)cyclopropane carboxylic acid from the sodium salt and the liberated 2,2-dimethyl-3-(2,2-dichlorovinyl)-cyclopropane carboxylic acid was extracted with methyl isobutyl ketone. The liquid extract was concentrated and evaporated to dryness, thereby 0.404 g of almost chemically pure 2,2-dimethyl-3-(2,2-dichlorovinyl)cyclopropane carboxlic acid (permethric acid) was obtained.

After 5 mg of the above 2,2-dimethyl-3-(2,2-dichlorovinyl)cyclopropane carboxylic acid was dissolved in 1 ml of toluene, equal molar amount each of thionyl chloride, pyridine and 3,5-dichloroaniline were added and allowed to react to produce anilide which was analyzed to determine the relative concentrations of the isomers of 2,2-dimethyl-3-(2,2-dichlorovinyl)cyclopropane carboxylic acid by high performance liquid chromatography (column: SUMIPAX OA-2100, moving phase: n-hexane/dichloroethane = 17/3(V/V), flow rate: 1.0 ml/min.). The results are shown in the following table, in which the yield represents the molar yield of (+)-trans-2,2-dimethyl-3(2,2-dichlorovinyl)cyclopropane carboxylic acid to ethyl (+)-trans-2,2-dimethyl-3-(2,2-dichlorovinyl)cyclopropane carboxylate contained in the starting material.

| Yield, | Ratio of the isomers of the resulting permethric acid |
|---|---|
| (%) | (+)-trans isomer/(-)-trans isomer/(+)-cis isomer/(-)-cis isomer |
| 100 | 90.1 : 9.9 : 0 : 0 |

### Example 5

Bacillus sp. DC-1 was cultivated in the same manner as in Example 4 and 0.5 g (wet weight) of cells was obtained from 100 ml of the cultured solution by centrifugation. After the harvested cells were suspended in 10 ml of 0.1 M phosphate buffer solution (pH 8.0), 1.0 g of methyl (±)-cis, trans-2,2-dimethyl-3-(2,2-dichlorovinyl)-cyclopropane carboxylate (cis/trans ratio = 45/55) was added to the suspension and the mixture was stirred at 30°C for 96 hours. Extraction and isolation from the resulting mixture was performed in the same manner as in Example 4 thereby 0.277 g of 2,2-dimethyl-3-(2,2-dichlorovinyl)cyclopropane carboxylic acid was obtained. All of the starting methyl 2,2-dimethyl-3-(2,2-dichlorovinyl)-cyclopropane carboxylate were recovered as it was excluding that which had been converted to 2,2-dimethyl-3(2,2-dichlorovinyl)cyclopropane carboxylic acid.

Analysis was effected in the same manner as in EXample 4 and the following results were obtained. In the table, the yield represents the molar yield of (+)-trans-2,2-dimethyl-3-(2,2-dichlorovinyl)cyclopropane carboxylic acid to methyl (+)-trans-2,2-dimethyl-3-(2,2-dichlorovinyl)cyclopropane carboxylate contained in the starting material.

| Yield | Ratio of the isomers in permethric acid |
|---|---|
| (%) | (+)-trans isomer/ (-)-trans isomer/ (+)-cis-isomer/ (-)-cis isomer |
| 100 | 93.0 : 7.0 : 0 : 0 |

### Examples 6 to 13

After 100 ml of a liquid medium (pH 6.5, prepared by dissolving 5.0 g of peptone, 10.0 g of glucose, 3.0 g of malt extract and 3.0 g of yeast extract in 1 ℓ of water) was put in a 500 ml flask and sterilized, a loopful of cells of the microorganism each shown in the Table 1 was inoculated from slant culture into the sterilized liquid medium and was subjected to shaking culture at 30°C for 20 hours. To the cultured solution was added 1.0 g of ethyl (±)-cis, trans-2,2-dimethyl-3(2,2-dichlorovinyl)cyclopropane carboxylate (cis /trans ratio = 45/55) and the mixture was reciprocally shaked at 30°C for 72 hours. Extraction, isolation and analysis of the resulting culture were performed in the same manner as in Example 4 to obtain almost chemically pure 2,2-dimethyl-3-(2,2-dichlorovinyl)-cyclopropane carboxylic acid (permethric acid). All of the ethyl 2,2-dimethyl-3-(2,2-dichlorovinyl)cyclopropane carboxylate added were recovered as it was excluding that which had been converted to 2,2-dimethyl-3-(2,2-dichlorovinyl)cyclopropane carboxylic acid. The analytical results of the reaction performed by each bacterial strain are shown in Table 1. In the table, the yield represents the molar yield of (+)-trans-2,2-dimethyl-3-(2,2-dichlorovinyl)cyclopropane carboxylic acid to ethyl (+)-trans-2,2-dimethyl-3-(2,2-dichlorovinyl)cyclopropane carboxylate contained in the starting material.

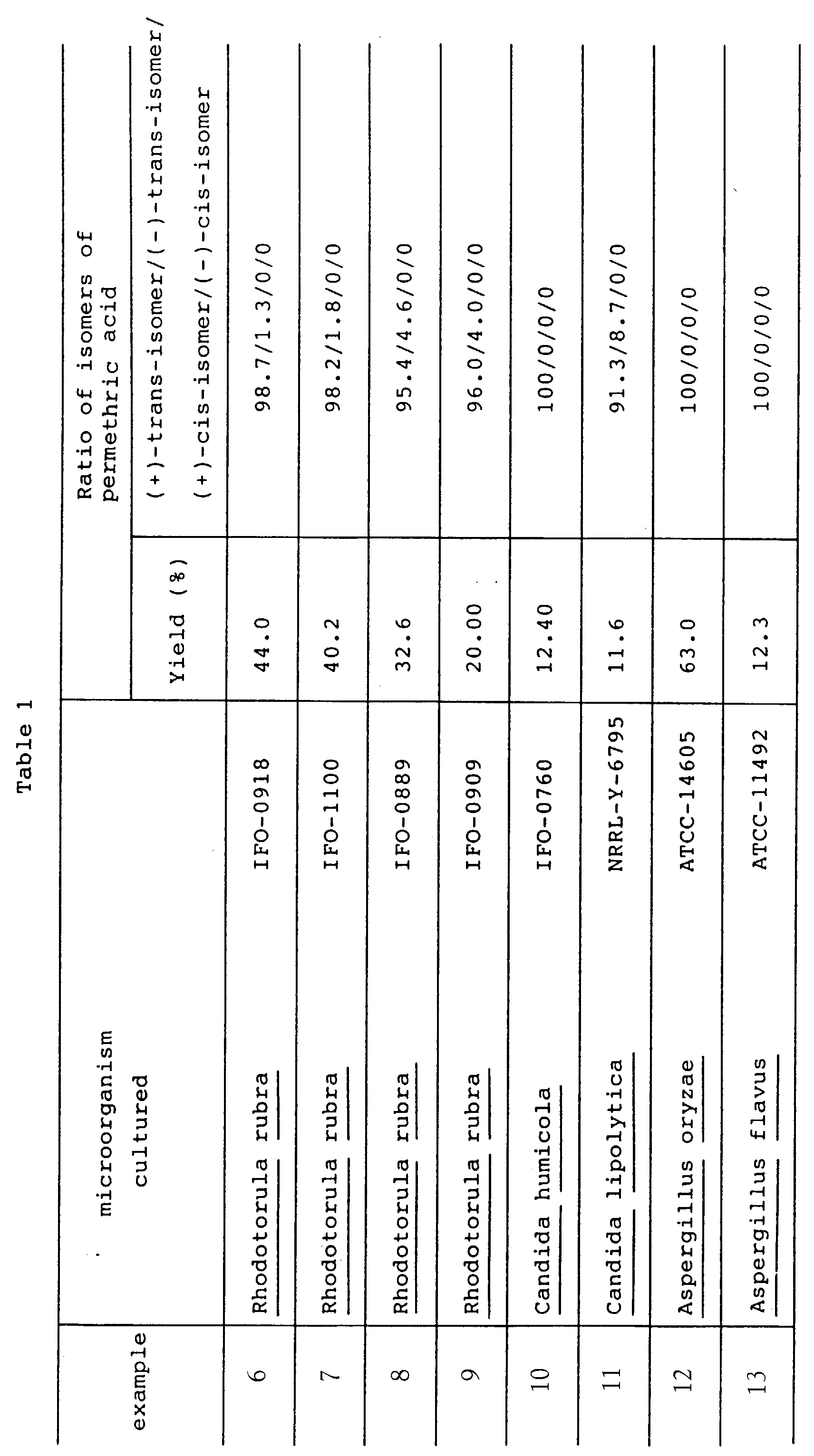

## Claims

1. A process for producing (+)-trans-2,2-dimethyl-3-(2,2-dichlorovinyl)cyclopropane carboxylic acid or its salts, which comprises allowing (±)-cis,trans-2,2-dimethyl-3-(2,2-dichlorovinyl)cyclopropane carboxylic acid ester represented by the formula (II): 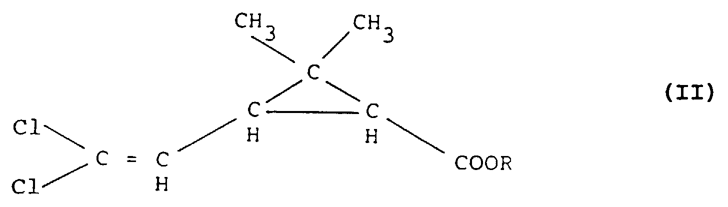 wherein R represents C₁-C₄ alkyl group or C₁-C₄ alkyl group substituted by halogen atom, which does not indicate the configuration of the ester, to react with
| | |
|---|---|
| Arthrobacter globiformis | IFO-12958 |
| Thermomyces lanuginosus | IFO-9863 |
| Rhodotorula rubra | IFO-0918 |
| Rhodotorula rubra | IFO-1100 |
| Rhodotorula rubra | IFO-0889 |
| Rhodotorula rubra | IFO-0909 |
| Candida humicola | IFO-0760 |
| Candida lipolytica | NRRL-Y-6795 |
| Aspergillus oryzae | ATTC-14605 |
| Aspergillus flavus | ATTC-11492 or |
| Bacillus sp. DC-1 (BP-FERM 1254) | |
or esterase produced by the above microorganisms, to asymmetrically hydrolyze the ester into (+)-trans-2,2-dimethyl-3-(2,2-dichlorovinyl)cyclopropane carboxylic acid and the esters of its stereoisomers and then recovering the resulting (+)-trans-2,2-dimethyl-3-(2,2-dichlorovinyl)cyclopropane carboxylic acid or salts thereof.

2. A process according to claim 1 wherein the ester of formula (II) is reacted with
Arthrobacter globiformis IFO-12958
or esterase produced thereby.

3. A process according to claim 1 wherein the ester of formula (II) is reacted with
Thermomyces lanuginosus IFO-9863
or esterase produced thereby.

4. A process according to claim 1 wherein the ester of formula (II) is reacted with
Rhodotorula rubra IFO-0918
or esterase produced thereby.

5. A process according to claim 1 wherein the ester of formula (II) is reacted with
Rhodotorula rubra IFO-1100
or esterase produced thereby.

6. A process according to claim 1 wherein the ester of formula (II) is reacted with
Rhodotorula rubra IFO-0889
or esterase produced thereby.

7. A process according to claim 1 wherein the ester of formula (II) is reacted with
Rhodotorula rubra IFO-0909
or esterase produced thereby.

8. A process according to claim 1 wherein the ester of formula (II) is reacted with
Candida humicola IFO-0760
or esterase produced thereby.

9. A process according to claim 1 wherein the ester of formula (II) is reacted with
Candida lipolytica NRRL-Y-6795
or esterase produced thereby.

10. A process according to claim 1 wherein the ester of formula (II) is reacted with
Bacillus sp. DC-1 (BP-FERM 1254)
or esterase produced thereby.

## Patentansprüche

1. Verfahren zur Herstellung von (+)-trans-2,2-Dimethyl-3-(2,2-dichlorvinyl)cyclopropancarbonsäure oder eines Salzes davon, umfassend die Umsetzung von (±)-cis, trans-2,2-Dimethyl-3-(2,2-dichlorvinyl)cyclopropancarbonsäureester der Formel (II) 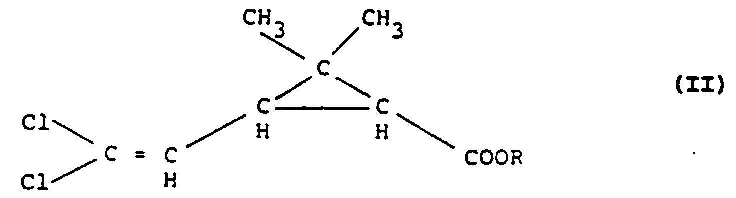 in der R einen C₁-C₄-Alkylrest oder einen mit einem Halogenatom substituierten C₁-C₄-Alkylrest bedeutet, wobei die Formel nicht die Konfiguration des Esters angibt, mit
| | |
|---|---|
| Arthrobacter globiformis | IFO-12958 |
| Thermomyces lanuginosus | IFO-9863 |
| Rhodotorula rubra | IFO-0918 |
| Rhodotorula rubra | IFO-1100 |
| Rhodotorula rubra | IFO-0889 |
| Rhodotorula rubra | IFO-0909 |
| Candida humicola | IFO-0760 |
| Candida lipolytica | NRRL-Y-6795 |
| Aspergillus oryzae | ATTC-14605 |
| Aspergillus flavus | ATTC-11492 oder |
| Bacillus sp. DC-1 (BP-FERM 1254) | |
oder einer von diesen Mikroorganismen erzeugten Esterase für die asymmetrische Hydrolyse des Esters zur Herstellung von (+)-trans-2,2-Dimethyl-3-(2,2-dichlorvinyl)cyclopropancarbonsäure und der Ester ihrer Stereoisomeren, und anschließende Gewinnung der (+)-trans-2,2-Dimethyl-3-(2,2-dichlorvinyl)cyclopropancarbonsäure oder von Salzen davon.

2. Verfahren nach Anspruch 1, wobei der Ester der Formel (II) mit
Arthrobacter globiformis IFO-12958
oder einer davon erzeugten Esterase umgesetzt wird.

3. Verfahren nach Anspruch 1, wobei der Ester der Formel (II) mit
Thermomyces lanuginosus IFO-9863
oder einer davon erzeugten Esterase umgesetzt wird.

4. Verfahren nach Anspruch 1, wobei der Ester der Formel (II) mit
Rhodotorula rubra IFO-0918
oder einer davon erzeugten Esterase umgesetzt wird.

5. Verfahren nach Anspruch 1, wobei der Ester der Formel (II) mit
Rhodotorula rubra IFO-1100
oder einer davon erzeugten Esterase umgesetzt wird.

6. Verfahren nach Anspruch 1, wobei der Ester der Formel (II) mit
Rhodotorula rubra IFO-0889
oder einer davon erzeugten Esterase umgesetzt wird.

7. Verfahren nach Anspruch 1, wobei der Ester der Formel (II) mit
Rhodotorula rubra IFO-0909
oder einer davon erzeugten Esterase umgesetzt wird.

8. Verfahren nach Anspruch 1, wobei der Ester der Formel (II) mit
Candida humicola IFO-0760
oder einer davon erzeugten Esterase umgesetzt wird.

9. Verfahren nach Anspruch 1, wobei der Ester der Formel (II) mit
Candida lipolytica NRRL-Y-6795
oder einer davon erzeugten Esterase umgesetzt wird.

10. Verfahren nach Anspruch 1, wobei der Ester der Formel (II) mit
Bacillus sp. DC-1 (BP-FERM 1254)
oder einer davon erzeugten Esterase umgesetzt wird.

## Revendications

1. Un procédé de production de l'acide (+)-trans-2,2-diméthyl-3(2,2-dichlorovinyl) cyclopropane-carboxylique ou de ses sels, qui consiste à faire réagir un ester d'acide (±)-cis,trans-2,2-diméthyl-3(2,2-dichlorovinyle) cyclopropane-carboxylique représenté par la formule (II): 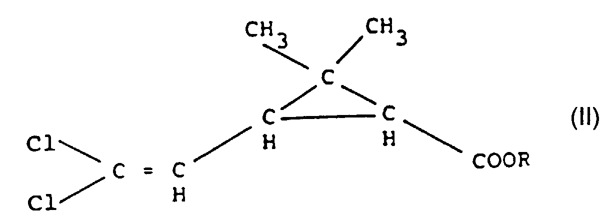 dans laquelle R représente un groupe alkyle ayant de 1 à 4 atomes de carbone ou un groupe alkyle ayant de 1 à 4 atomes de carbone substitué par un atome halogène, qui n'indique pas la configuration de l'ester, sur 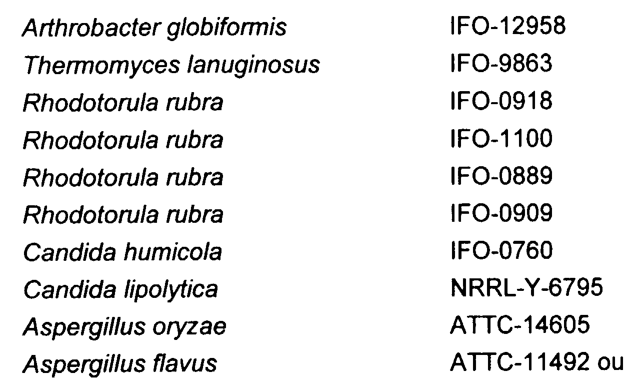 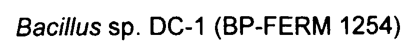 ou une estérase produite par les micro-organismes ci-dessus, de manière à hydrolyser de façon asymétrique l'ester en acide (+)-trans-2,2- diméthyl-3(2,2-dichlorovinyl) cyclopropane-carboxylique et les esters de ses stéréoisomères et ensuite à recueillir l'acide (+)-trans-2,2- diméthyl-3(2,2-dichlorovinyl) cyclopropane-carboxylique obtenu et les sels de celui-ci.

2. Un procédé selon la revendication 1 dans lequel on fait réagir l'ester de formule (II) sur
*Arthrobacter globiformis* IFO-12958
ou une estérase produite par ce micro-organisme

3. Un procédé selon la revendication 1 dans lequel on fait réagir l'ester de formule (II) sur
*Thermomyces lanuginosus* IFO-9863
ou une estérase produite par ce micro-organisme.

4. Un procédé selon la revendication 1 dans lequel on fait réagir l'ester de formule (II) sur
*Rhodotorula rubra* IFO-0918
ou une estérase produite par ce micro-organisme.

5. Un procédé selon la revendication 1 dans lequel on fait réagir l'ester de formule (II) sur
*Rhodotorula rubra* IFO-1100
ou une estérase produite par ce micro-organisme.

6. Un procédé selon la revendication 1 dans lequel on fait réagir l'ester de formule (II) sur
*Rhodotorula rubra* IFO-0889
ou une estérase produite par ce micro-organisme.

7. Un procédé selon la revendication 1 dans lequel on fait réagir l'ester de formule (II) sur
*Rhodotorula rubra* IFO-0909
ou une estérase produite par ce micro-organisme.

8. Un procédé selon la revendication 1 dans lequel on fait réagir l'ester de formule (II) sur
*Candida humicola* IFO-0760
ou une estérase produite par ce micro-organisme.

9. Un procédé selon la revendication 1 dans lequel on fait réagir l'ester de formule (II) sur
*Candida lipolytica* NRRL-Y-6795
ou une estérase produite par ce micro-organisme.

10. Un procédé selon la revendication 1 dans lequel on fait réagir l'ester de formule (II) sur
*Bacillus* sp. DC-1 (BP-FERM 1254)
ou une estérase produite par ce micro-organisme.
